# EUROPEAN PATENT APPLICATION

(11) **EP 4 796 167 A1**
(43) Date of publication of application: **26.08.2026**
(21) Application number: 25382171.4
(22) Date of filing: 25.02.2025
(51) Int. Cl.: A61N 2/00, A61B 5/055, A61B 5/369, A61N 2/02

(54) **SYSTEM AND METHOD FOR APPLYING TARGETED AND OPTIMAL TRANSCRANIAL MAGNETIC STIMULATION TREATMENT**

(71) Applicant: Connectoma Neurotech SL, 28006 Madrid (ES)
(72) Inventor: Diez García, Daniel, Madrid (ES); Tames Llana, Alfonso, Madrid (ES); Adán Manes, Jaime, Madrid (ES)
(74) Representative: Pons IP

(57) **Abstract**

A system and method for identifying the maximum anticorrelation point between dorsolateral prefrontal cortex (DLPFC) and Subgenual Anterior Cingulate Cortex (sgACC), identifying the motor hotspot, calculating the motor threshold (MT), predicting Transcranial Magnetic Stimulation (TMS) intensity, and ensuring real-time precision in TMS treatment using multimodal functional Magnetic Resonance Imaging (fMRI) and Electroencephalography (EEG) data, Electromyography (EMG) data, neuronavigator devices, and a robotized support. The system leverages AI architectures to process and integrate fMRI and EEG data, enabling precise localization of the target for TMS treatment of depression and addictions, and personalized determination of TMS intensity, and TMS sessions scheduling and duration parameters. The integration with neuronavigator and robotized hardware systems ensures real-time adjustments to maintain accurate stimulation targeting (location and tilt) despite patient head movements or coil misalignments. The invention improves the precision, safety, and efficacy of TMS targeting, leading to better outcomes for patients with depression and addictions.

## Description

### OBJECT OF THE INVENTION

The present invention relates to the field of neuroimaging, artificial intelligence (AI), neuromodulation, and robotics. Specifically, it pertains to a system and method for identifying the an optimal stimulation point and optimal TMS intensity for specific protocols, and ensuring real-time spatial precision in Transcranial Magnetic Stimulation (TMS) treatment for depression and addictions (nicotine addiction, alcohol use disorder, cocaine and stimulant addiction, opioid addiction, cannabis use disorder, gambling and food addiction, binge eating disorder, screen addiction, compulsive shopping, fitness and exercise addiction and social media addiction).

The invention allows to maintain accurate stimulation targeting and treatment parameters calculation and real-time readjustments in case of patient head movements or coil misalignments.

### BACKGROUND OF THE INVENTION

Depression and addictions are leading causes of disability worldwide. TMS is a non-invasive neuromodulation technique used to treat depression and addictions by targeting specific brain regions, such as the dorsolateral prefrontal cortex (DLPFC). Clinical studies suggest that this same type of calculation and treatment could have optimal results and provide an alternative to the use of drugs in the case of other conditions such as post-traumatic disorder, addiction treatment or obsessive-compulsive disorder, due to its role in mood regulation, executive function, decision-making, and impulse control.

The subgenual anterior cingulate cortex (sgACC) is a key brain region that seems to be hyperactivated in both depression and addictions, leading to hyperactivation of the default mode network (DMN), which is associated with depressive symptoms and addictive behaviors.

The motor threshold (MT) is a critical parameter for personalizing TMS treatment intensity. It is determined by stimulating the primary motor cortex (M1) and measuring the minimum intensity required to elicit a muscle response. The MT is defined by the minimum intensity of stimulation that elicits a measurable response (either visually or via electromyogram (EMG)) in at least 50% of delivered pulses to the target muscle, usually the abductor pollicis brevis muscle.

Neuronavigator devices and robotized TMS systems are used to ensure precise targeting of brain regions during TMS.

Current TMS targeting methods rely on anatomical landmarks or standardized brain atlases, which do not account for individual variability in functional connectivity or cortical excitability. Additionally, they lack real-time adjustments for patient movements.

### DESCRIPTION OF THE INVENTION

The object of the present invention focuses on a system and method for obtaining an optimal target treatment point, preferably being the point in the dorsolateral prefrontal cortex (DLPFC) with maximum functional anticorrelation with the Subgenual Anterior Cingulate Cortex (sgACC), and a personalized TMS intensity for treating a neurological disease; and for applying TMS using those parameters in an automated way.

The system of the invention comprises:
- A data acquisition module configured to acquire a dataset comprising at least anatomical MRI images, fMRI images and EEG data from a patient.
- A processing unit connected to the data acquisition module and configured to obtain a target treatment point, preferably a maximum anticorrelation point between the DLPFC and the sgACC, integrating anatomical features extracted from the anatomical MRI images, functional features extracted from the fMRI images and source activity maps obtained by performing source localization techniques on the EEG data, and to obtain a personalized TMS intensity by applying a predefined percentage of the motor threshold (MT), depending on the selected protocol.

- A robotized TMS module connected to the TMS guidance module and comprising:
   - A TMS coil.
   - A robotized support configured to support and move the TMS coil;
- One or more motion tracking sensors configured to measure patient's head position and TMS coil position and tilt.
- A neuronavigator module, connected to the processing unit and comprising a neuronavigator device configured to define a stimulation position of the TMS coil for stimulating the target treatment point.
- A TMS guidance module connected to the tracking sensors, to the neuronavigator module and to the robotized TMS module; and configured to receive the measurements of the patient's head position, the TMS coil position and tilt and the stimulation position of the TMS coil and to define an updated stimulation position based on the movement of the patient's head.

The robotized support is configured to move the TMS coil to the stimulation position of the TMS coil, and when changes about patient's head position and/or tilt are detected, to the updated stimulation position.

The invention also describes a method for applying targeted and optimal TMS treatment, comprising the steps of:
- Acquiring a dataset comprising anatomical MRI images, functional Magnetic Resonance Imaging (fMRI) images and Electroencephalography (EEG) data from a patient.
- extracting anatomical features from the anatomical MRI images using a 3D neural network to determine structural information about the brain;
- extracting functional features from fMRI images using a 3D neural network for determining brain activity and connectivity information;
- Extracting temporal features from EEG data using a 1D neural network.
- performing source localization techniques using the temporal features from EEG data for determining source activity maps representing electrical activity on each brain region;
- Identifying a target treatment point by integrating the anatomical features and the functional features with the source activity maps.
- Identifying a primary motor cortex.
- Estimating a motor threshold (MT) as minimum stimulation intensity in the primary motor cortex producing muscle response.
- Determining a personalized TMS intensity as a predefined percentage, often (but not only) between the range of 90-120% of the MT.
- Estimating a stimulation position for the TMS treatment coil to reach the target treatment point.
- Moving the TMS coil automatically to the stimulation position using the robotized support.
- Measuring patient's head position and TMS coil position and tilt using tracking sensors.
- Updating the stimulation position of the TMS coil using the robotized support when changes about patient's head position and/or tilt are detected.

Preferably, the anatomical features are selected from: cortical thickness, gray matter density, skull thickness, distance to target, sulcal and gyral patterns and brain volume.

In some preferred embodiments, the method of the invention could further comprise a step of normalizing brain MRI images and fMRI images by using Automated Anatomical Labeling (AAL) with Harvard-Oxford brain atlas or Yeo brain atlas, extracting, for each region, an average time series of the BOLD (Blood Oxygen Level Dependent) signal across all voxels within said region.

Also, the method could comprise also a step of preprocessing the fMRI data by applying slice timing correction, motion correction, spatial normalization to an anatomical brain image, smoothing, temporal filtering, with a bandpass filter in the range of 0.01-0.1 Hz, and/or regression of nuisance variables, for removing signals selected from white matter, cerebrospinal fluid, and motion parameters.

Preferably, the functional features are selected from: functional connectivity maps, anticorrelation maps, regional activation, network metrics, degree centrality, clustering coefficient, betweenness centrality and spatial patterns of activity.

The step of extracting functional features from fMRI images could be performed by calculating pairwise correlation, partial correlation, mutual information, spectral coherence and/or granger causality.

Regarding the temporal features extracted from the EEG, they preferably could be selected from frequency band power, event-related potentials and temporal patterns.

The 1D neural network could be trained to detect artifacts, learn and filter frequency-specific bands related to the artifacts responses and map a specific frequency signal to a brain region.

Moreover, said 1D neural network could be combined with transformers for prioritizing relevant features.

Preferably, the step of performing source localization techniques could be performed by calculating source localization maps, frequency-specific spatial maps or spatial-temporal connectivity maps.

The step of identifying a target treatment point by integrating the anatomical features and the functional features with the source activity maps could be preferably performed using an attention-based fusion or a concatenation mechanism to prioritize relevant information.

The target region selected could be the maximum anticorrelation point between DLPFC and sgACC or a specific region responsible for controlling a specific muscle in the primary motor cortex.

The method could also comprise a step of validating the step of identifying a target treatment point, being said validation step performed by using cross-validation and clinical ground truth.

Preferably, the step of identifying a primary motor cortex could be performed using finger tapping during the fMRI acquisition.

The motor threshold estimation could also be performed using artificial intelligence models based on anatomical features selected from: distance to motor cortex (M1), cortical excitability, skull thickness over M1 and anatomical variability.

More preferably, the motor threshold (MT) estimation could be performed using machine Learning Models selected from: Random Forests, Gradient Boosting Machines, SVMs, and/or Deep Learning Models selected from: CNNs, RNNs or Transformers.

Also, the step of estimating the MT could be performed by detecting an elicited muscle response or using an electromyogram (EMG).

The method of the present invention could further comprise a step of simulating an electric field distribution of the stimulation in the brain based on individual head anatomy derived from MRI data and the coil position, orientation, and the TMS intensity. The simulation could be performed using Finite Element Modeling (FEM).

Also, the method could comprise a step of iteratively adjusting the coil position, orientation, and the TMS intensity, based on the electric field distribution simulated, for achieving a predefined stimulation in the target point while minimizing stimulation of non-target areas.

More preferably, the TMS intensity could be adjusted by using reinforcement learning algorithms with a reward function based on maintaining a motor evoked potential measured within a predetermined range.

In preferred embodiments, the TMS intensity adjusted is simulated before applying.

### DESCRIPTION OF THE DRAWINGS

To complement the description that is being made and for the purpose of helping to better understand the features of the invention according to a preferred practical exemplary embodiment thereof, a set of drawings is attached as an integral part of said description in which the following is depicted in an illustrative and non-limiting manner:
Figure 1 shows a diagram showing the architecture of the method for applying targeted and optimal TMS treatment, based on the main steps.
Figure 2 shows a diagram representing the detailed steps of the method for applying targeted and optimal TMS treatment.

### PREFERRED EMBODIMENT OF THE INVENTION

The present invention provides a system and method for method for applying targeted and optimal TMS treatment, identifying the maximum anticorrelation point between dorsolateral prefrontal cortex (DLPFC) and the subgenual anterior cingulate cortex (sgACC), predicting optimal Transcranial Magnetic Stimulation (TMS) intensity, and ensuring real-time precision in TMS treatment using multimodal functional Magnetic Resonance Imaging (fMRI) and Electroencephalography (EEG) data, an electromyogram (EMG), a neuronavigator module, a TMS guidance module and a robotized TMS module.

The system and method leverage AI architectures (e.g., Random Forests, Convolutional Neural Networks (CNN), Transformers, Reinforcement Learning, and Bayesian Neural Networks) to process and integrate fMRI and EEG data, enabling precise localization of a target for TMS treatment of depression and addictions. The integration with the neuronavigator module, the TMS guidance module, the robotized TMS module and one or more tracking sensors ensures real-time adjustments to maintain accurate stimulation targeting (position and tilt) despite patient head movements or coil misalignments.

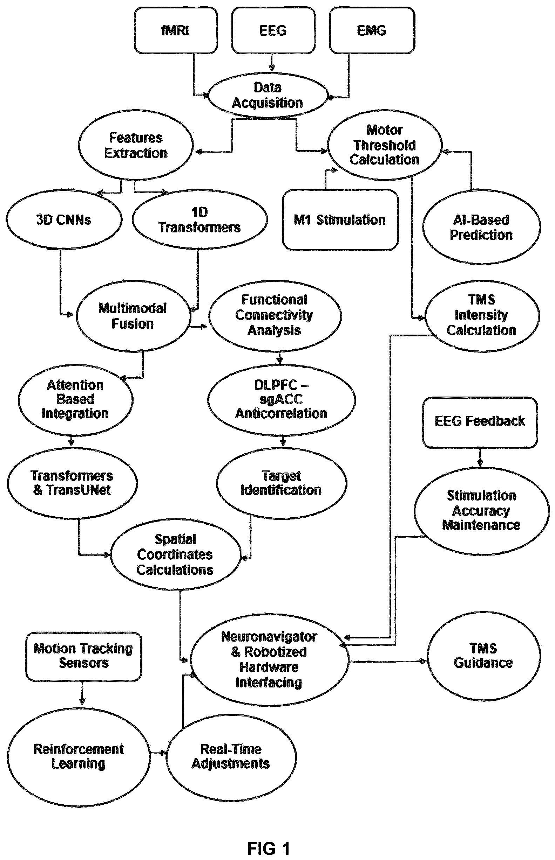

The main steps of the method herein described are represented in the diagram of Figure 1.

Firstly, a data acquisition module is responsible for acquiring anatomical images, fMRI images and EEG data from the patient. All this data is then pre-processed. In the case of fMRI, this pre-processing is done to remove noise and artifacts by performing one or more of:
- Slice Timing Correction: Adjust for differences in acquisition time across slices.
- Motion Correction: Align volumes to correct for head motion.
- Spatial Normalization: Warp the brain images to the standard MNI template.
- Smoothing: Apply spatial smoothing to improve signal-to-noise ratio.
- Temporal Filtering: Apply a bandpass filter in the range within 0.01-0.1 Hz to remove low-frequency drift and high-frequency noise.
- Regression of Nuisance Variables: Remove signals from white matter, cerebrospinal fluid, and motion parameters.

Also, to be compliant with a standardized brain reference model, the method of the invention could use AAL, Harvard-Oxford or Yeo brain atlas. The brain image normalization is performed to MRI images and fMRI images for the time series and using the atlas brain regions. Thus, for each region it is extracted average time series of the BOLD (Blood Oxygen Level Dependent) signal across all voxels within said region.

EEG data is also pre-processed to remove artifacts and filter relevant frequency bands.

Later steps are carried out by a processing unit which comprises: a feature extraction module, a motor threshold calculation module, a multimodal fusion module, a target identification module and a TMS Intensity Determination Module.

After that, by means of the feature extraction module, this pre-processed data, then undergoes feature extraction, wherein fMRI data is processed using a 3D TransUNet to extract functional features and compute anticorrelation maps. TransUNet is a hybrid model that combines Transformers and U-Net (a CNN) for medical image segmentation.

The 3D neural network is used to extract spatial features from the MRI images and the fMRI images. In terms of spatial features coverage:
In the context of the invention, the spatial features refer to the characteristics extracted from anatomical MRI, EEG, MEG and functional MRI (fMRI) data that help identify and localize brain regions of interest at the dorsolateral prefrontal cortex (DLPFC) and subgenual anterior cingulate cortex (sgACC). These features are critical for determining the maximum anticorrelation point between these regions and for guiding the TMS treatment.

Particularly, the spatial features could be:
1. Anatomical Features: derived from the anatomical MRI images and provide structural information about the brain, being:
   - Cortical Thickness: Measures the thickness of the cortical gray matter in specific regions (e.g., DLPFC, sgACC).
   - Gray Matter Density: Quantifies the density of gray matter in target regions, which is relevant for cortical excitability.
   - Skull Thickness: Influences the attenuation of the TMS magnetic field and is used to adjust stimulation intensity.
   - Distance to Target: The Euclidean distance between the TMS coil and the target brain region (e.g., DLPFC or sgACC).
   - Sulcal and Gyral Patterns: Identifies the folds (gyri) and grooves (sulci) of the brain, which are used to localize functional regions.
   - Brain Volume: Measures the volume of specific brain regions or subregions (e.g., prefrontal cortex, anterior cingulate cortex).
2. Functional Features: derived from the fMRI data and provide information about brain activity and connectivity, being:
   - Functional Connectivity Maps: Represent the statistical dependencies (e.g., correlations) between brain regions, such as the DLPFC and sgACC.
   - Anticorrelation Maps: Highlight regions with negatively correlated activity.
   - Regional Activation: Measures the level of activation in specific brain regions during rest or task-based fMRI.
   - Network Metrics: Quantifies properties of brain networks, such as:
   - Degree Centrality: Number of connections to a region.
   - Clustering Coefficient: Measure of local connectivity.
   - Betweenness Centrality: Importance of a region in information flow.
   - Spatial Patterns of Activity: Identifies spatial patterns of activity associated with specific cognitive or emotional states (e.g., default mode network activation).

The extraction of functional features is performed by calculating correlation connectivity matrixes within the brain regions using the below mechanisms:
- **Pairwise Correlation:** Calculate the Pearson correlation coefficient between the time series of each pair of ROIs. This results in a correlation matrix (N x N, where N is the number of ROIs).
- **Partial Correlation:** Controls for the influence of other ROIs.
- Mutual Information: Measures non-linear dependencies.
- **Spectral Coherence:** Measures frequency-specific connectivity.
- **Granger Causality:** Assesses directional influence between regions.

In the case of EEG data, it is processed using a 1D neural network to extract temporal features and map signals to brain regions, the extracted temporal features could be:
- Frequency band power (e.g., alpha, beta, gamma).
- Event-related potentials (ERPs).
- Temporal patterns associated with specific cognitive or emotional states.

The input to the 1D neural network is the EEG time series data, which is inherently one-dimensional (1D) because it represents electrical activity over time at each electrode.

The EEG time series data is preprocessed by removing artifacts (e.g., eye blinks, muscle activity), and filtering frequency bands.

In EEG (Electroencephalography) data, specific frequencies (e.g., alpha, beta, gamma) are associated with different brain states or cognitive processes, but they do not directly point to a specific brain area in isolation. This is because EEG measures electrical activity from the scalp, which reflects the summed activity of millions of neurons across large brain regions. It is required to combine frequency analysis with source localization techniques, to estimate the brain regions generating specific frequency bands and inference and decouple the contribution of each of the regions. 1D models of this invention are trained to calculate the scenarios of overlapping based not only on the lectures of one of the EEG sensors (but in the total group) and establish the most probable regions activation levels combination to complete the whole view.

Source localization is the process of estimating the origins of electrical activity recorded by EEG sensors on the scalp. As explained, EEG measures the summed electrical activity of millions of neurons and it is challenging to pinpoint the exact brain regions generating these signals. Source localization solves this "inverse problem" by using mathematical models and algorithms to map scalp-recorded EEG signals to their underlying brain sources.

Next, the Functional Connectivity Analysis Module computes functional connectivity maps such as DLPFC-sgACC anticorrelation from fMRI data and performs source localization on the EEG data to map the signals to brain regions.

The 1D neural network learns to map the extracted temporal features to specific brain regions by training on labeled data (being simultaneous EEG-fMRI or ground truth source locations). The output of the network is a set of source activity maps, which represent the estimated electrical activity in different brain regions.

By using the 1D neural network, the following advantages are obtained:
- **Temporal Resolution:** Captures the dynamic nature of EEG signals.
- **Efficiency:** Processes time series data directly without requiring complex transformations.
- **Adaptability:** Can be trained to handle individual variability in brain anatomy and function.

Then, the source localization results (the mapped brain regions using a standardized brain atlas to stablish a reference model) are integrated with the fMRI functional features to compute functional connectivity maps and identify the maximum anticorrelation point between the DLPFC and sgACC. This integration ensures precise targeting for TMS treatment.

The integration between the source localization results and the fMRI functional features is performed using multimodal fusion, which combines spatial features from fMRI with temporal features from EEG to improve target localization.

Alternatively, the integration between the source localization results and the fMRI functional features could be used to stablish a sequence of points to stimulate at the TMS session to cover an anticorrelation point or area.

Said integration provides combined spatial-temporal features such as:
- Source Localization Maps: Maps EEG signals to specific brain regions using inverse solutions (such sLORETA, beamforming).
- Frequency-Specific Spatial Maps: Identifies brain regions associated with specific EEG frequency bands (such alpha, beta, gamma).
- Spatial-Temporal Connectivity: Combines fMRI spatial connectivity with EEG temporal dynamics to identify functional networks.

Then, in the next step an identification of the Primary Motor Cortex (M1) is performed using a combination of the anatomical MRI images, the functional MRI (fMRI) images, and machine learning models. These models help to precisely locate for example a hand knob area or other relevant regions within M1 that are responsible for controlling specific muscles.

Then, the motor threshold calculation module combines anatomical features (e.g., skull thickness, distance to M1), functional features (such cortical excitability), machine learning models and experimental measurements (e.g., EMG) to estimate the motor threshold.

In this case, the motor threshold (MT) estimation uses the following spatial features:
- Distance to Motor Cortex (M1): The distance between the TMS coil and the primary motor cortex.
- Cortical Excitability: Measures the responsiveness of the motor cortex to stimulation, derived from fMRI, EMG or EEG data.
- Skull Thickness Over M1: Influences the intensity of stimulation required to elicit a motor response.
- Anatomical Variability: Individual differences in the location and size of the motor cortex.

The motor threshold is determined by stimulating the primary motor cortex (M1) and measuring the resulting muscle responses. This could be done using Electromyography (EMG), which records the electrical activity of muscles (e.g., in the hand) in response to cortical stimulation.

The MT is defined as the minimum stimulation intensity required to elicit a predefined muscle response (e.g., a motor evoked potential, MEP) in a specified percentage of trials (e.g., 50% of the time).

In some embodiments, simulation software like SimNIBS could be used to calculate the intensity in the TMS coil that produces the same electric field in the area of interest as that produced during the motor threshold measurement. This integration of experimental and computational approaches ensures precise, personalized, and effective brain stimulation.

The simulation process involves the following steps:
∘ Simulating an electric field distribution in the brain derived from an TMS intensity application using finite element modelling (FEM) based on individual head anatomy derived from MRI data, the TMS coil position, orientation, and stimulation intensity.
∘ Determining the intensity in the antenna (or coil) that produces the same electric field in the area of interest as that produced during the motor threshold (MT) measurement. For example, if the motor threshold corresponds to an electric field strength of 100 V/m in M1, the simulation can iteratively adjust the coil current in the simulation until the desired field strength is achieved. This ensures precise and personalized stimulation.
∘ The simulated electric field is validated against experimental data (historical EMG measurements) to ensure accuracy. If discrepancies are found, the head model or stimulation parameters are adjusted to improve the match by integrating the execution corrections as qualified data feedback for the data acquisition system. This approach enables the optimization of stimulation protocols, ensuring that the desired electric field is achieved in the target region while minimizing stimulation of non-target areas.

Thus, the spatial features used to adjust TMS intensity and coil position in real time are:
- Electric Field Distribution: the electric field strength in the target region.
- Head Movement Metrics: Spatial changes in head position and tilt, measured by motion tracking sensors.
- Coil Position and Orientation: Spatial coordinates and orientation of the TMS coil relative to the target region.

In some embodiments, machine Learning Models, such as Random Forests, Gradient Boosting Machines, SVMs; and Deep Learning Models, such as CNNs, RNNs or Transformers, are used to predict the motor threshold (MT) based on features like skull thickness, cortical excitability, and distance to the motor cortex.

In the following step, the multimodal fusion module integrates the extracted fMRI spatial features and EEG temporal features using an attention-based fusion or concatenation mechanism to prioritize relevant information. To do so a Transformer-based fusion module models the interactions between fMRI and EEG data.

Then, the target identification module predicts the spatial coordinates of the maximum anticorrelation point between the DLPFC and the sgACC using the integrated fMRI and EEG features as well as the integrated outcomes from this invention AI models and the adaptive response to the real-time EEG and EMG monitored captured data. The output is validated using cross-validation and clinical ground truth.

In the next step, the TMS Intensity Determination Module calculates a personalized TMS intensity based on the calculated motor threshold (MT). The intensity for the magnetic pulse is adjusted using a predefined percentage of the MT depending on the selected protocol which, in this case, ranges from 110-120%.

In some embodiments, the motor threshold calculation and stimulation process can be enhanced by incorporating a reinforcement learning (RL) model to enable real-time adaptation of TMS intensity during treatment.

This approach allows the system to dynamically adjust stimulation parameters based on ongoing feedback, ensuring optimal and personalized treatment outcomes.

The RL model aims to maximize the effectiveness of TMS by maintaining the desired motor response (e.g., motor evoked potentials, MEPs) while minimizing side effects or overstimulation.

Thus, the RL model initializes with the patient's baseline motor threshold and initial TMS intensity. During treatment, the model continuously monitors the current stimulation intensity, EMG signals (measured muscle response) and adjusts the TMS intensity based on the reward function. Then, the adjusted intensity is validated using simulations to ensure the electric field in the target region remains within the desired range. This process is repeated in real time, ensuring optimal stimulation throughout the treatment session.

The RL is defined by an action: adjustment of TMS intensity (increasing or decreasing the coil current); and a reward: based on the quality of the motor response and the consistency of the response with the desired outcome.

Thus, a high reward is given if the MEP amplitude is within the target range and a penalty is applied if the MEP amplitude is too low (ineffective stimulation) or too high (overstimulation).

In this way, if the MEP amplitude decreases below the target range, the RL model may increase the stimulation intensity. Conversely, if the MEP amplitude exceeds the target range, the RL model may reduce the intensity.

Also, if the RL model increases the coil current, the simulation process could be used to verify that the resulting electric field remains within safe and effective limits.

Using the RL model provides clear advantages such as:
∘ Personalization: The RL model tailors the stimulation intensity to the individual patient's anatomy and responses, improving treatment efficacy.
∘ Dynamic Adjustment: The system adapts to changes in the patient's neural activity or motor threshold during the treatment session.
∘ Optimization: The RL model optimizes stimulation parameters to achieve the desired therapeutic effect while minimizing side effects.

In some embodiments, this step could use a reinforcement learning model to implement for real-time adaptation of TMS intensity during treatment.

In the following step, the neuronavigator module takes the previously identified target, defined by its spatial coordinates, to map said target to the patient's brain in real time. During this step, the patient's head position is tracked in real time using multiple motion tracking sensors.

The previously obtained data: predicted coordinates and personalized intensity are used to provide a guidance for TMS targeting in real time. Simultaneously, EEG data can be obtained and used during TMS to provide real time feedback about TMS treatment efficacy.

Using the information about patient's head position, the target coordinates and the neuronavigator device, the method uses a robotized TMS module comprising a TMS coil and a robotized support that positions the TMS coil in a determined position and tilt based on the predicted target coordinates. This coil position is dynamically adjusted position in response to patient head movements or coil misalignments.

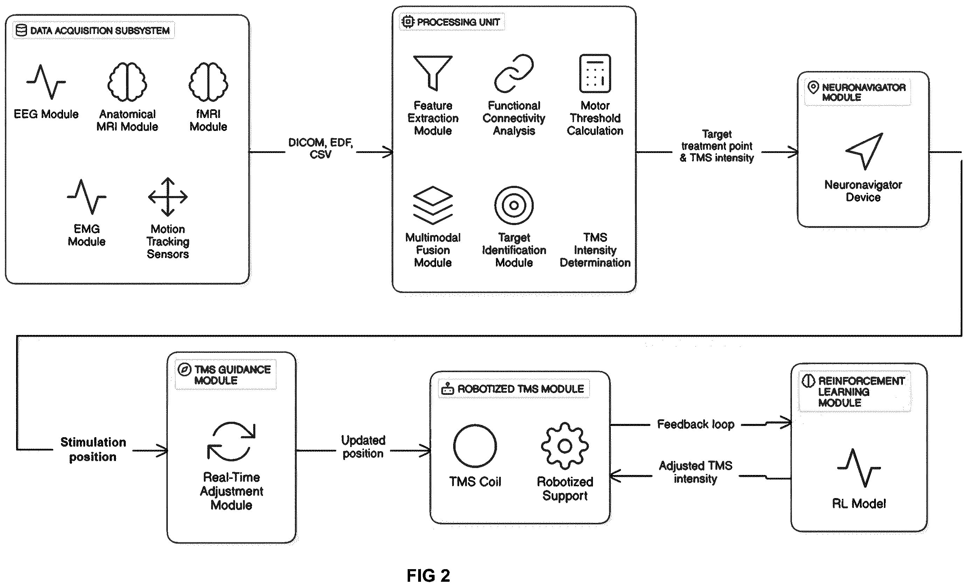

The previously detailed steps are represented in the diagram of Figure 2.

## Claims

1. A system for applying a Transcranial Magnetic Stimulation (TMS) treatment, comprising:
- a data acquisition module configured to acquire a dataset comprising anatomical MRI images, fMRI images and EEG data from a patient;
- a processing unit connected to the data acquisition module and configured to obtain a a target treatment point integrating anatomical features extracted from the anatomical MRI images, functional features extracted from the fMRI images and source activity maps obtained by performing source localization techniques on the EEG data, and to obtain a personalized TMS intensity by applying a predefined percentage;
- a robotized TMS module comprising:
• a TMS coil and;
• a robotized support configured to support the TMS coil and to move said TMS coil;
- one or more motion tracking sensors configured to measure patient's head position and TMS coil position and tilt;
- a neuronavigator module, connected to the processing unit and comprising a neuronavigator device configured to define a stimulation position of the TMS coil for stimulating the target treatment point;
- a TMS guidance module connected to the tracking sensors, to the neuronavigator module and to the robotized TMS module; and configured to receive the measurements of the patient's head position, the TMS coil position and tilt and the stimulation position of the TMS coil and to define an updated stimulation position based on the movement of the patient's head;
wherein the robotized support is configured to move the TMS coil to the updated stimulation position.

2. A method for applying targeted and optimal TMS treatment, comprising the steps of:
- acquiring a dataset comprising anatomical MRI images, functional Magnetic Resonance Imaging (fMRI) images and Electroencephalography (EEG) data from a patient;
- extracting anatomical features from the anatomical MRI images using a 3D neural network to determine structural information about the brain;
- extracting functional features from fMRI images using a 3D neural network for determining brain activity and connectivity information;
- extracting temporal features from EEG data using a 1D neural network;
- performing source localization techniques using the temporal features from EEG data for determining source activity maps representing electrical activity on each brain region;
- identifying a target treatment point by integrating the anatomical features and the functional features with the source activity maps;
- identifying a primary motor cortex;
- estimating a motor threshold (MT) as minimum stimulation intensity in the primary motor cortex producing muscle response;
- determining a personalized TMS intensity as a predefined percentage;
- estimating a stimulation position for the TMS treatment coil to reach the target treatment point;
- moving the TMS coil automatically to the stimulation position using the robotized support;
- measuring patient's head position and TMS coils position and tilt using tracking sensors;
- updating the stimulation position of the TMS coil using the robotized support when changes about patient's head position and/or tilt are detected.

3. The method according to claim 2, wherein the anatomical features are selected from: cortical thickness, gray matter density, skull thickness, distance to target, sulcal and gyral patterns and brain volume.

4. The method according to any of claims 2 to 3, further comprising an step of normalizing brain MRI images and fMRI images by using Automated Anatomical Labeling (AAL) with Harvard-Oxford brain atlas or Yeo brain atlas, extracting, for each region, an average time series of the BOLD (Blood Oxygen Level Dependent) signal across all voxels within said region.

5. The method according to any of claims 2 to 4, further comprising an step of preprocessing the fMRI data by applying slice timing correction, motion correction, spatial normalization to an anatomical brain image, smoothing, temporal filtering, with a bandpass filter in the range of 0.01-0.1 Hz, and/or regression of nuisance variables, for removing signals selected from white matter, cerebrospinal fluid, and motion parameters.

6. The method according to any of claims 2 to 5, wherein the functional features are selected from: functional connectivity maps, anticorrelation maps, regional activation, network metrics, degree centrality, clustering coefficient, betweenness centrality and spatial patterns of activity.

7. The method according to any of claims 2 to 6, wherein the step of extracting functional features from fMRI images is performed by calculating pairwise correlation, partial correlation, mutual information, spectral coherence and/or granger causality.

8. The method according to any of claims 2 to 7, wherein the temporal features extracted from the EEG are selected from frequency band power, event-related potentials and temporal patterns.

9. The method according to any of claims 2 to 8, wherein the 1D neural network is trained to detect artifacts, learn and filter frequency-specific bands related to the artifacts responses and map a specific frequency signal to a brain region.

10. The method according to any of claims 2 to 9, wherein the step of performing source localization techniques is performed by calculating source localization maps, frequency-specific spatial maps or spatial-temporal connectivity maps.

11. The method according to any of claims 2 to 10, wherein the step of identifying a target treatment point by integrating the anatomical features and the functional features with the source activity maps is performed using an attention-based fusion or a concatenation mechanism to prioritize relevant information.

12. The method according to any of claims 2 to 11, wherein the target region is the maximum anticorrelation point between dorsolateral prefrontal cortex (DLPFC) and Subgenual Anterior Cingulate Cortex (sgACC) or a specific region responsible for controlling a specific muscle in the primary motor cortex.

13. The method according to any of claims 2 to 12, wherein the motor threshold is estimated by:
- using artificial intelligence models based on anatomical features selected from: distance to motor cortex (M1), cortical excitability, skull thickness over M1 and anatomical variability;
- detecting an elicited muscle response;
- or through an electromyogram (EMG).

14. The method according to any of claims 2 to 13, further comprising a step of simulating an electric field distribution of the stimulation in the brain based on individual head anatomy derived from MRI data and the coil position, orientation, and the TMS intensity.

15. The method according to claim 14, further comprising a step of iteratively adjusting the coil position, orientation, and the TMS intensity, based on the electric field distribution simulated and using reinforcement learning algorithms with a reward function based on maintaining a motor evoked potential measured within a predetermined range, for achieving a predefined stimulation in the target point while minimizing stimulation of non-target areas.
